(19)

Europäisches
Patentamt
European
Patent Office
Office européen
des brevets

(11) **EP 2 264 988 A1**

(12) **EUROPEAN PATENT APPLICATION**

(43) Date of publication:
   **22.12.2010 Bulletin 2010/51**

(51) Int Cl.:
   **H04M 1/725** (2006.01)

(21) Application number: **09163077.2**

(22) Date of filing: **18.06.2009**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK TR**<br>Designated Extension States:<br>**AL BA RS** | • **Technische Universität Berlin**<br>**10623 Berlin (DE)**<br><br>(72) Inventor: **Ketabdar, Hamed**<br>**10713 berlin (DE)**<br><br>(74) Representative: **Vossius & Partner**<br>**Siebertstrasse 4**<br>**81675 München (DE)** |
| (71) Applicants:<br>• **Deutsche Telekom AG**<br>**53113 Bonn (DE)** | |

(54) **Method of detecting a current user activity and environment context of a user of a mobile phone using an accelerator sensor and a microphone, computer program product, and mobile phone**

(57)    The invention provides a method of detecting a current user activity and environment context of a user of a mobile phone comprising at least one acceleration sensor and a microphone, the method comprising the steps of obtaining at least one acceleration signal from said at least one acceleration sensor, obtaining an audio signal from said microphone, said audio signal representing environmental sound of the mobile phone, pre-processing said obtained at least one acceleration signal and pre-processing said obtained audio signal to remove redundant information present in said signals, performing a feature extraction on said pre-processed at least one acceleration signal, performing a feature extraction on said pre-processed audio signal; and classifying user activity and environment context based on the acceleration pattern represented by the extracted acceleration features and on the audio pattern represented by the extracted audio features.

Fig. 1

EP 2 264 988 A1

## Description

Field of the Invention

The invention provides a method of detecting a current user activity and environment context of a user of a mobile phone, a computer program product, and a mobile phone. The present invention particularly relates to techniques for using embedded accelerometer, microphone, and computational resources in a mobile phone for recognizing a subject (user of the mobile phone) activity and environment context.

Background of the invention

Over the past few years, many mobile phones are equipped with new sensors such as acceleration sensors. The acceleration pattern can be different, during different activities such as walking, being seated, being in a car, etc. In addition, all mobile phones can collect audio information through embedded microphone which can give some ideas about the ongoing activities/context such as being in a meeting, in a party, in street, etc.

Human activity classification and context sensing has been attempted with cameras, microphones, inertial sensors, temperature, ambient light, and proximity sensors, etc. Researchers have developed custom algorithms based on each sensor or a combination of sensors to classify human activity and context. In these known approaches, usually a group of sensors are worn by a subject or carried in a unit. The data collected by the sensors are then transmitted to a central processing unit to be processed by the activity classification algorithm.

US-A-7 421 369 is related to an apparatus, method, program and storage medium for activity recognition. The apparatus includes a sensor unit with linear and rotational motion sensors to capture linear and rotational motions, and a computational unit configured to receive and process signals fo sensors. US-A-7 421 369 is primarily concerned about measuring different body segment activities rather than classification of activities in certain groups and does not particularly talk about using a mobile phone for such a purpose.

US-A-2002/167 488 discloses a mobile device which uses at least one sensor for capturing contextual information, and uses this contextual information for responding to incoming messages and notifications. However, it only talks about context sensing with application in responding to messages and notifications.

DE-A-10 2005 019 924 presents an activity measurement device for assessing activity of experimental subjects, in which the device is attached to body of experimental subject. The device is arranged on a belt clip on the body of the experimental subject and uses acceleration sensors.

JP-2003-219 061 describes a mobile phone that uses an acceleration sensor to sense an impact force, a microphone to detect a shock sound, and a GPS signal to detect a current position, and transmits an emergency message in case a traffic accident is detected by means of the accelerator and the microphone.

Summary of the Invention

According to a first aspect, the invention provides a method of detecting a current user activity and environment context of a user of a mobile phone comprising at least one acceleration sensor and a microphone, the method comprising the steps of:

obtaining at least one acceleration signal from said at least one acceleration sensor;
obtaining an audio signal from said microphone, said audio signal representing environmental sound of the mobile phone;
pre-processing said obtained at least one acceleration signal and pre-processing said obtained audio signal to remove redundant information present in said signals;
performing a feature extraction on said pre-processed at least one acceleration signal;
performing a feature extraction on said pre-processed audio signal; and
classifying user activity and environment context based on the acceleration pattern represented by the extracted acceleration features and on the audio pattern represented by the extracted audio features.

According to the invention, all the sensory and computational units are integrated in a mobile phone which makes the invention user friendly, inexpensive and easy to access or spread for users. Unlike related work on human activity detection, it does not impose the burden of wearing or carrying additional devices and/or sensors.

The step of classifying user activity and environment context preferably comprises comparing said acceleration pattern and said audio pattern with statistical reference models for different activities. The statistical model is preferably an artificial neural network or a Gaussian mixture model. Alternatively, the step of classifying user activity and environment context comprises comparing said acceleration pattern and said audio pattern with a Hidden Markov model, wherein each state of the model models an activity and context class.

The features extracted from said pre-processed at least one acceleration signal are preferably selected from the group comprising: acceleration magnitude over different axes, the rate of change in acceleration over different axis, the absolute magnitude of the acceleration, and pairwise difference between acceleration magnitudes over different axis. Moreover, the features extracted from said pre-processed audio signal are preferably selected from the group comprising: magnitude, loudness, energy over different frequency bands, and periodicity or pitch.

According to a preferred embodiment, the classification step further comprises the step of integrating predetermined time and duration information about activities.

It is preferred that the method further comprises the step of changing an operating state of the mobile phone depending on the classification result. More preferably, applications and/or functionalities running on the mobile phone are newly prioritized to adapt the mobile phone to the detected user activity and enviromnent context.

The invention relates to a mobile phone. Preferably, the mobile phone ring is turned on or off or the volume is changed to adapt the mobile phone to the detected user activity and environment context.

According to a preferred aspect, the method further comprises the step of comparing the detected user activity and environment context with a regular user activity and environment context of the user, which may have applications in health care. This preferred embodiment of the invention will be described in more detail below.

According to a second aspect, the invention provides a mobile phone comprising at least one acceleration sensor providing at least one acceleration signal to a processor; and a microphone for providing an audio signal representing environmental sound of the mobile phone to said processor. The processor is configured for pre-processing the at least one acceleration signal and for pre-processing the audio signal to remove redundant information present in the signals. The processor is further configured for performing a feature extraction on the pre-processed at least one acceleration signal and for performing a feature extraction on the pre-processed audio signal; and is configured for classifying user activity and environment context based on the acceleration pattern represented by the extracted acceleration features and on the audio pattern represented by the extracted audio features.

Preferably, additional sensors such as proximity and ambient light sensors are used for determining the user activity and environment context.

Although the invention is described in relation to a mobile phone, other devices such as palm, handheld, or PDAs are also encompassed by the invention.

Thus, in more general terms, the invention provides a method using accelerometer sensor(s) and a microphone integrated in a mobile phone for detecting current activity of the user and environment context. The acceleration sensor and microphone output are first pre-processed to remove redundant information and extract features which represent the user activity in a discriminative way. These features are used to create reference statistical models for different activities. The outcome of the statistical models is then used in a decision tree along with some given information about the duration and timing of daily activities in order to detect current activity and context. This allows recognizing and distinguishing certain activities such as walking, sitting, being in a meeting, working, being in a party, sleeping, etc, or the physical and/or medical condition of the user.

During the test of the system, actual samples of microphone and acceleration data (after feature extraction) are presented to the trained reference models. A score is estimated based on the match between ongoing samples and reference models for different activities. This score is used as a basis for classification of different activities.

The present invention encompasses several applications of detecting user activity and context. According to one preferred embodiment, it is used to automatically turn on/off mobile phone ring or change the ring volume, and/or prioritize some applications and functionalities of the mobile phone depending on the current activity and situation of the user. According to an alternative embodiment, the activity detection is used in taking care of elderly and children. It can help to monitor their activities, and optionally provides a medical doctor with a summary of daily physical activities, as well as warnings in case of unexpected movements (moving disorders, falling, accident, etc.). It can be also used to study the progress in physical movements after a surgery, and related E-health issues.

Thus, the present invention proposes new approaches to solve the problem of subject activity and context classification, and prevent the mentioned limitations of the state of the art techniques. The invention uses acceleration sensor(s) embedded in a mobile as well as microphone audio input to classify the activity of user and environment context. Pre-processing and classification of data is also done using the computational resources in the mobile phone. Therefore, in the present invention, there is no need for wearing special sensors on the body, or carrying extra sensory and hardware units. The present invention tries to solve the activity and context classification problem with a sophisticated data processing rather than demanding a sophisticated and expensive hardware. Since a mobile phone is usually carried by the subject in normal daily life, it does not impose a big burden or change in the habits of the subject. In contrast, some of the mentioned state of the art techniques proposing to wear sensors on the body or carry special device can be unusual, unfriendly and frustrating for the subject. In addition, the present invention extends the application of the subject activity classification and context sensing beyond only controlling the functionalities in a mobile phone. It proposes to use a portable device such as a mobile phone which is able to classify user activities and context, for the purpose of monitoring different activities (such as walking, sitting, sleeping, being in a meeting, working, etc.), surveillance and security (to detect unusual movements such as falling, car accident), clinical medical research, E-health, and taking care of children and elderly. As an additional option, since a mobile phone is equipped with radio link facilities, all the collected information can be optionally sent to a central system for further detailed analysis. However, this is only an additional option, and data analysis and activity classification can be fully accomplished on the mobile phone hardware.

The invention is advantageous and superior to known implementations because it fits into a regular mobile phone, and is able to operate with the limited resources available in the mobile phone in terms of sensory and hardware components. It does not impose designing or using a new hardware for activity classification. It also does not impose the burden of carrying or wearing an additional unit or even additional units on the body. It is based on a mobile phone which is regularly carried by the subject.

According to further aspects of the invention, the concept of analysing user activity and context is implemented in various applications based on a mobile phone which is able to classify user activity and context.

As mentioned before, one preferred application is prioritizing or modifying mobile phone functionalities based on the ongoing activity or context. For example, the mobile phone ring is switched off or changed to vibration mode if it is detected by the phone that the user is sleeping, in a meeting, in a car, etc. Another example could be providing easier access to calendar when is detected by the phone that the user is at work or in a meeting, e.g., by putting the calendar shortcut on the main screen/menu. If it is detected by the phone that the user is in a party, the mobile phone can provide easier access to camera application, again by enabling camera option in the main menu or main screen. The user can additionally set the mobile phone to do certain custom actions or launch certain applications in case of detecting specific context or activity class. For instance, the user can choose to have his incoming calls diverted to another number in case of certain activities.

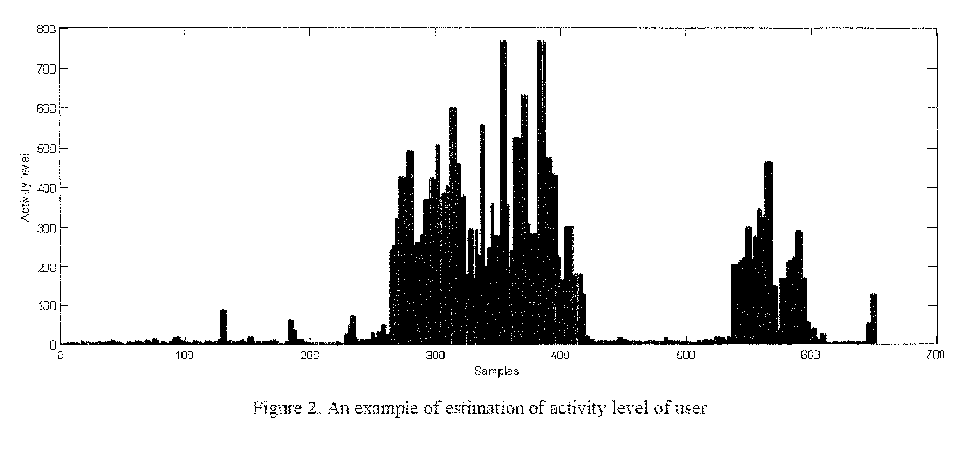

A mobile phone equipped with activity/context analysis according to the invention is preferably used for monitoring and taking care of elderly and children. It detects/classifes their current activity or situation and sends this information to a designated person for monitoring. This person (or an automatic application) then checks if they are in the expected situation at a certain time of day. For instance, the mobile phone sends reports about ongoing activity of the user (elderly and children) to a monitoring person or automatic center. The monitoring system is configured to check, for example, if the user has not been moving (walking) for a long time, or has been moving for long time, and to issue warnings in case of unexpected scenarios. As an alternative, raw data of acceleration and audio can be uploaded in a server to be observed by a monitoring agent. The agent can check the level of physical activity of a user or several users over time. Fig. 2 shows an example for estimation of activity level for a user over time. In order to estimate activity level, the difference between consecutive peaks and valleys is measured and presented. The agent can check the activity level of several users in the same time. In order to facilitate monitoring several users, the server can use the same method explained before to classify user activities, and issue warnings to the agent in case of risky activities for a certain user. The agent can then check the activity level for that user more precisely, and decide about helping him.

A mobile phone equipped with activity/context analysis according to the invention is preferably used as a user friendly and convenient device for E-health related issues, for example to manage a disease state or physical condition of a user. Many diseases affect the pattern of certain physical activities such as walking. A mobile phone equipped with activity classification and analysis can observe the pattern of certain activities for a user, and compare it with normal pattern for the same activity. The mobile phone informs the user about any deviation form the normal pattern, and issues warnings about early detection of symptoms of a certain disease. Since the mobile phone is equipped with radio links, this information is preferably sent to a designated medical doctor or other health care professionals for further analysis. Such a mobile phone can be also used for checking the progress of a patient after certain surgeries.

Instead of sending information by mobile communication, the invention also encompasses other wireless techniques, e.g., infrared (IR) microwaves, or Bluetooth, or other optical techniques, to transmit information for example to a PDA, laptop computer, or desktop computer for further evaluation of the detected signals. Thus, the invention is preferably protocol-independent.

As another application related to E-health, the mobile phone according to the invention is preferably used for observing energy/calorie consumption of a user. The mobile phone analyses the amount of user's physical activity, excluding situations such as riding a car, or being in a bus or metro, and come up with an estimation of energy/calorie consumption over a certain interval. Moreover, the mobile phone is preferably configured to allow the user to input caloric content of foods eaten, and may further estimate calorie consumption during an exercise performed. In this way, e. g., a person in a weight-loss program may see in great detail whether they are expending more calories in the form of exercise than the same individual is consuming in the form of food. In all of these respects, the portable aspect of the device is important: to wit, the user may conveniently carry the device, e.g. the mobile phone on their person wherever they may go, allowing data processing at the time needed.

Other aspects, features, and advantages will be apparent from the summary above, as well as from the description that follows, including the figures and the claims.

The invention will now be described with reference to the accompanying drawings which show in

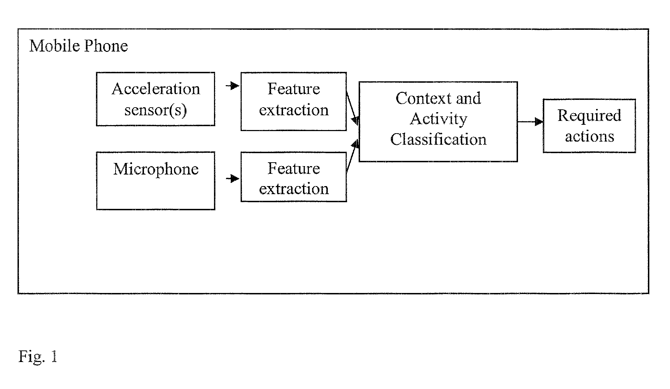

Fig. 1    a general overview of the system according to the invention;

Fig. 2    a diagram showing an example of estimation of activity level of a user;

Fig. 3 an example of raw measured data for acceleration along x, y, and z-axis;

Fig. 4 for the example of Fig. 3, the pre-processed acceleration signals;

Fig. 5 an example of raw measured data for audio signal; and

Fig. 6 for the example of Fig. 5, the pre-processed audio signal.

The term "user" as used herein primarily indicates a user of a mobile phone. The same may be a medical patient under physician care, a person interested in maintaining health via accurate recording of nutrition and exercise, and so on. The term "patient" is used, in addition to a person under the care of a physician, to also refer to a "normal" or healthy individual who is interested in maintaining a healthy physiologic balance.

According to the invention, data is captured using at least one acceleration sensor and a microphone integrated in the mobile phone. The acceleration sensor raw data and microphone raw data are stored in a buffer memory, pre-processed by digital filters to remove noise, and used to extract features which represent acceleration and audio modalities more discriminatively. Extracted features are then used in a context and activity classification component to classify the ongoing scenario. In addition to features, some prior knowledge such as duration and timing is optionally integrated in the classification process. It should be noted that the feature extraction and classification modules are also implemented using mobile phone computational resources. The acceleration sensor(s) is(are) integrated in the mobile phone and captures linear acceleration in x, y and z directions. The microphone is the regular microphone integrated in the mobile phone and is used according to the invention to capture audio information.

The feature extraction modules receiving the detected signals from the acceleration sensor(s) and the microphone disregard redundant information and provide a modified or refined representation of acceleration and audio signals which is more discriminative for classifying ongoing activity and context.

Acceleration based features are features extracted from the accelerometer data and are mainly acceleration magnitude over different axis, the rate of change in acceleration (over different axis), the absolute magnitude of the acceleration, and pairwise difference between acceleration magnitudes over different axis.

Audio based features are features extracted from the audio signal and are mainly based on magnitude (or loudness), energy over different frequency bands, and periodicity or pitch.

In the next step, activity and context classification is performed. Different activities have different acceleration and audio pattern signature associated with them. The classification module receives features extracted from audio and acceleration data, and builds statistical reference models for different activities during a training phase. The training phase can be done at a provider company, and do not need to be necessarily done by the final user. During the training phase, several feature samples of each context or activity class are presented to the statistical model. The statistical model can be an artificial neural network or Gaussian mixture models. Parameters of the statistical model are trained according to the samples presented for each context class, in order to cover the feature space corresponding to each class. Such a trained model can then be used to estimate scores for different context/activity classes during testing the system. As the simplest strategy, the class having the highest score is selected as ongoing activity or context.

In order to capture the information existing over time or sequence of context/activities, the statistical model can be replaced with a Hidden Markov model (HMM). In this case, each state of the HMM models a certain context/activity class, and transition probabilities between different states represent the possibilities for transition between different context/activity classes. For instance, the 'working' activity should be preceded usually by 'walking' activity, as the user usually needs to walk out his house to reach work. In addition, heuristically designed binary decision trees can be used on top of the classification results to further smooth out the decisions about ongoing activity/context by integrating some prior knowledge related to duration and timing of different context/activities.

The statistical reference models are optionally adapted for a certain user. This adaptation can provide more accurate models for different activities of a certain user, and subsequently better accuracy in detecting the user activity and context. The adaptation of the statistical models can be based on MAP (Maximum A Posteriori) or other relevant statistical adaptation techniques.

Extra prior information such as duration or time of certain activities can be integrated in decision making (classification) process, Certain physical activities can have certain maximum or minimum duration. For instance, sleeping may last a few hours, meetings may last between 30 minutes to two hours, etc. In addition, there can be certain correlation between physical activities and time. For instance, walking is mostly to happen in the morning when the user wakes up and goes to work, at noon, when the user goes for lunch, and in the afternoon when the user goes home. Meeting and sitting activities are most probable during the work time, say between Sam to 6pm. All these timing and duration prior information can be integrated in the decision making process, and possibly provide more accurate activity classification results.

Information about user habits can be also integrated in decision making process. For instance, the algorithm can lean the pattern and timing of certain physical activities during the weekdays and weekends, and use this information to predict ongoing physical activity more accurately.

According to a preferred embodiment of the invention, extra information such as location information (provided by GPS, Wi-Fi, radio towers) is additionally integrated in the decision making process. Thus, it is possible to associate different physical activities with certain locations. For instance, a meeting can happen at a location defined as work place, or sleeping can happen at the residence (home). This extra location information helps to have more accurate decision on activity and context detection.

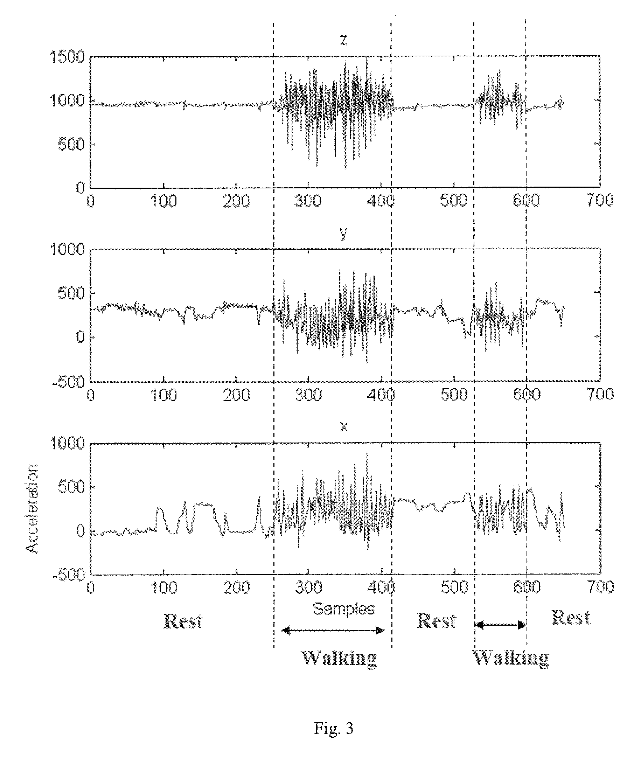

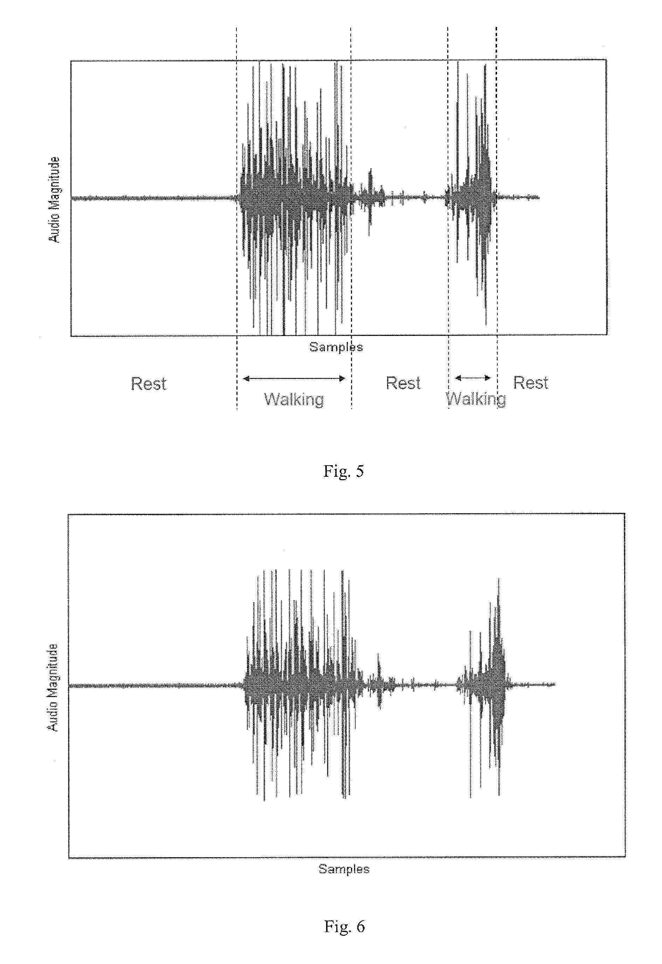

Fig. 3 shows raw samples of acceleration along x, y, and z axis over time for an example according to the invention. The acceleration samples are recorded during two different activities, namely walking and resting. The mobile phone is carried by the user in his pant pocket during recording. As marked in the figure, there is a discriminative difference between the pattern of acceleration during different activities. The corresponding audio pattern is shown in Fig. 5. Also in this Figure the discriminative difference between the pattern of acceleration during different activities can be seen.

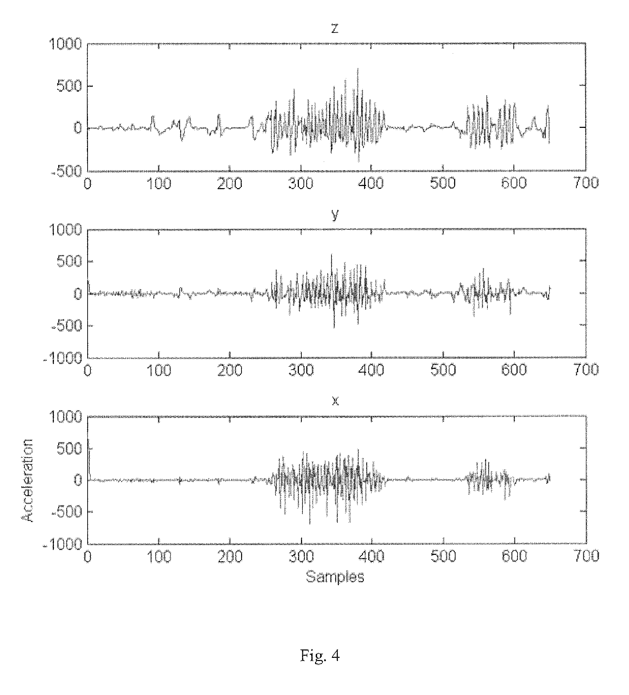

According to the invention, pre-processing is performed on the measured raw data. Regarding pre-processing, the method which is applied is different based on the application. In general, the pre-processing step is usually a digital high pass or low pass filter (depending on the application). A sample of acceleration signals (the same signals as in Fig. 3) along x, y, and z axis after pre-processing with high pass filter are shown in Fig.4. Fig. 6 shows the audio pattern of Fig. 5 after being pre-processed.

After pre-processing, feature extraction is performed. According to the invention, feature extraction methods are used which are not computationally expensive considering limited computational resources available in a mobile phone. All the feature extraction methods are applied to a time window of either acceleration and audio signal. This window can be 1-2 seconds long. The value of extracted features is averaged over samples in each window. Adjacent windows can have overlaps up to 80%.

Acceleration based features:

The following 4 types of features are preferred according to the invention:

1. Acceleration magnitude over different axis: This is absolute value of acceleration over different x, y, and z axis, i.e. $|a_x|$, $|a_y|$,$|a_z|$, where a indicates acceleration

2. Rate of change of acceleration over different axis: This is derivate of acceleration signal (over different axis) with respect to time, i.e. $\dfrac{da_x}{dt}$, $\dfrac{da_y}{dt}$,

$\dfrac{da_z}{dt}$.

3. Absolute magnitude of acceleration: This is defined as: $a = \sqrt{a_x^2 + a_y^2 + a_z^2}$

4. Pairwise difference between acceleration magnitudes along different axis, i.e. $|a_x - a_y|$, $|a_x - a_z|$,$|a_y - a_z|$

However, it is also preferred that combinations or even all of these types are applied together.

Audio based features:

1. Magnitude: This is defined as average energy of the audio signal samples in a limited time window (1-2 seconds) around the current time sample.

2. Energy over different frequency bands: This is defined as average energy of audio signal samples in a limited window (1-2 seconds around current time sample) within a certain frequency range.

3. Periodicity or pitch: This is defined as fundamental frequency component of audio signal in a limited time window (1-2 seconds around current time sample).

Further details on extraction of audio based features can be found in Fundamental of Speech Recognition; Lawrence Rabiner & Biing-Hwang Juang Englewood Cliffs NJ: PTR Prentice Hall (Signal Processing Series), c1993, ISBN 0-13-015157-2.

In addition to above main features, some variants of them can be also used in feature extraction.

The process according to the present invention is preferably employed as follows. Once the mobile phone is switched on or even if the functionality according to the invention is initiated separately by the user, the acceleration sensor(s) measures the acceleration values. Furthermore, audio information is detected by the microphone. This may be performed continuously or intermittently. Furthermore, the sensors may be operated consecutively or in an alternating manner. A data processor of the device receives the detected signals from the individual sensors separately, and performs feature extraction. Based at least in part or entirely on the detected acceleration parameters and audio information, a filtered or "cleaned" acceleration and audio signals are calculated in that redundant information is removed from the signals, as also described above. These data are then supplied to the activity and context classification component of the processor. Here, the acceleration pattern and audio pattern are compared with statistical reference models stored in a memory. The result of such comparison process is the determination of a specific user activity pattern and environment context reflecting the current activity and environmental situation of the user.

In contrast to the prior art, such as US 2002/167488, the mobile phone according to the invention is used also for user activity classification in addition to the mere context sensing. Activity recognition can help in having a more advanced context sensing algorithm. Moreover, the invention provides several extended applications for activity and context sensing (such as monitoring, E-health, taking care of elderly and children, security, surveillance). Moreover, the method according to the invention is beneficial compared to, e.g., JP-2003-219 061 as it allows to distinguish between a sudden impact caused by a car accident and a sudden impact just caused by the user letting the mobile phone falling down on the ground because the environmental context is taken into account in the decision making process.

**Claims**

1.  Method of detecting a current user activity and environment context of a user of a mobile phone comprising at least one acceleration sensor and a microphone, the method comprising the steps of:

    obtaining at least one acceleration signal from said at least one acceleration sensor; and
    obtaining an audio signal from said microphone, said audio signal representing environmental sound of the mobile phone;
    **characterized by**
    pre-processing said obtained at least one acceleration signal and pre-processing said obtained audio signal to remove redundant information present in said signals;
    performing a feature extraction on said pre-processed at least one acceleration signal;
    performing a feature extraction on said pre-processed audio signal; and
    classifying user activity and environment context based on the acceleration pattern represented by the extracted acceleration features and on the audio pattern represented by the extracted audio features.

2.  The method of claim 1, wherein the step of classifying user activity and environment context comprises comparing said acceleration pattern and said audio pattern with statistical reference models for different activities.

3.  The method of claim 2, wherein said statistical model is an artificial neural network or a Gaussian mixture model.

4.  The method of claim 1, wherein the step of classifying user activity and environment context comprises comparing said acceleration pattern and said audio pattern with a hidden Markov model, wherein each state of the model models a activity and context class.

5.  The method of any of the preceding claims, wherein features extracted from said pre-processed at least one acceleration signal are selected from the group comprising: acceleration magnitude over different axes, the rate of change in acceleration over different axis, the absolute magnitude of the acceleration, and pairwise difference between acceleration magnitudes over different axis.

6.  The method of any of the preceding claims, wherein features extracted from said pre-processed audio signal are selected from the group comprising: magnitude, loudness, energy over different frequency bands, and periodicity or pitch.

7.  The method of any of the preceding claims, the classifying step further comprising the step of integrating predetermined time and duration information about activities.

8.  The method of any of the preceding claims, further comprising the step of changing an operating state of the mobile phone depending on the classification result.

9.  The method of claim 8, wherein applications and/or functionalities running on said mobile phone are newly prioritized or modified to adapt the mobile phone device to the detected user activity and environment context.

10. The method of claim 9, wherein the mobile phone ring is turned on or off or the volume is changed to adapt the mobile phone to the detected user activity and environment context.

11. The method of any of the preceding claims, further comprising the step of comparing the detected user activity and environment context with a regular user activity and environment context of the user.

12. Mobile phone comprising
    at least one acceleration sensor providing at least one acceleration signal to a processor; and
    a microphone for providing an audio signal to said processor;
    **characterized by**
    said processor being configured for pre-processing said at least one acceleration signal and for pre-processing said audio signal to remove redundant information present in said signals, being further configured for performing a feature extraction on said pre-processed at least one acceleration signal and for performing a feature extraction on said pre-processed audio signal; and being configured for classifying user activity and environment context based on the acceleration pattern represented by the extracted acceleration features and on the audio pattern represented by the extracted audio features.

13. Computer program product, comprising instructions for performing the method of any of claims 1 to 11.

14. A method for acquiring and monitoring health data from patients comprising:

(a) detecting a current user activity and environment context of a user with the user's mobile phone according to the method of any of claims 1 to 11;
(b) uploading data representing the detected current user activity and environment context of a user from the mobile phone over a network to a remote server for storage therein.

15. The method of claim 14, further comprising the step of evaluating the uploaded data for irregularities compared to expected activity and context data.

16. The method of claim 14, further comprising the step of analysing the detected activity and context data for patterns, and comparing the detected patterns with expected normal patterns in order to determine irregularities.

Fig. 1

Figure 2. An example of estimation of activity level of user

Fig. 3

Fig. 4

Fig. 5

Fig. 6

Europäisches
Patentamt

European
Patent Office

Office européen
des brevets

## EUROPEAN SEARCH REPORT

Application Number

EP 09 16 3077

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 916 828 A (SONY FRANCE SA [FR]) 30 April 2008 (2008-04-30) | 1-13 | INV. H04M1/725 |
| Y | * paragraphs [0001] - [0008], [0020] - [0065]; figures 1-3 * | 14-16 | |
| X | EP 1 333 650 A (NOKIA CORP [FI]) 6 August 2003 (2003-08-06) * paragraphs [0018] - [0165] * | 1-13 | |
| X | EP 1 732 300 A (SONY CORP [JP]) 13 December 2006 (2006-12-13) * paragraphs [0020] - [0063] * | 1-13 | |
| Y | WO 2009/055207 A (MEDTRONIC INC [US]; GOETZ STEVEN M [US]) 30 April 2009 (2009-04-30) * the whole document * | 14-16 | |
| A | US 2007/255509 A1 (LEFEBVRE WILLIAM [US] ET AL) 1 November 2007 (2007-11-01) * paragraphs [0058], [0062] - [0064] * | 1 | TECHNICAL FIELDS SEARCHED (IPC) |
| A | US 6 238 337 B1 (KAMBHATLA NANDA [US] ET AL) 29 May 2001 (2001-05-29) * column 5, line 63 - column 6, line 11 * | 1 | H04M G01P |

The present search report has been drawn up for all claims

| Place of search | Date of completion of the search | Examiner |
|---|---|---|
| Munich | 2 December 2009 | Agreda Labrador, A |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 09 16 3077

This annex lists the patent family members relating to the patent documents cited in the above-mentioned European search report.
The members are as contained in the European Patent Office EDP file on
The European Patent Office is in no way liable for these particulars which are merely given for the purpose of information.

02-12-2009

| Patent document cited in search report | | Publication date | Patent family member(s) | | Publication date |
|---|---|---|---|---|---|
| EP 1916828 | A | 30-04-2008 | CN 101203008 A<br>JP 2008113442 A | | 18-06-2008<br>15-05-2008 |
| EP 1333650 | A | 06-08-2003 | US 2004176958 A1 | | 09-09-2004 |
| EP 1732300 | A | 13-12-2006 | CN 1941752 A<br>JP 2006345269 A<br>KR 20060128737 A<br>US 2007112922 A1 | | 04-04-2007<br>21-12-2006<br>14-12-2006<br>17-05-2007 |
| WO 2009055207 | A | 30-04-2009 | NONE | | |
| US 2007255509 | A1 | 01-11-2007 | NONE | | |
| US 6238337 | B1 | 29-05-2001 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7421369 A
- US 2002167488 A
- DE 102005019924 A
- JP 2003219061 A

**Non-patent literature cited in the description**

- **Lawrence Rabiner ; Biing-Hwang Juang.** Fundamental of Speech Recognition. PTR Prentice Hall, 1993